Europäisches Patentamt

European Patent Office

Office européen des brevets

(11) Publication number: 0 367 537
A2

# EUROPEAN PATENT APPLICATION

(21) Application number: 89311192.2

(22) Date of filing: 30.10.89

(51) Int. Cl.5 **B01J 19/02 , C22C 9/06 , C22C 19/00**

(30) Priority: 31.10.88 JP 275458/88

(43) Date of publication of application:
09.05.90 Bulletin 90/19

(84) Designated Contracting States:
AT BE CH DE ES FR GB GR IT LI LU NL SE

(71) Applicant: POLYPLASTICS CO. LTD.
3-13, Azuchicho, 2-chome
Chuo-Ku Osaka-shi Osaka(JP)

(72) Inventor: Kakizaki, Hideo
885-11, Miyajima
Fuji-shi Shizuoka(JP)
Inventor: Nakamura, Hiroshi
O-ron Road Lane 60 Alley 2, No.1, F2
Kao Shion San Min(TW)
Inventor: Akiyama, Minoru
1-8-18 Imaizumi
Fuji-shi Shizuoka(JP)
Inventor: Akimoto, Atsushi
39-3 Gokanjima
Fuji-shi Shizuoka(JP)

(74) Representative: Jackson, Peter et al
HYDE, HEIDE & O'DONNELL 146 Buckingham
Palace Road
London SW1W 9TR(GB)

(54) Equipment and method for handling formaldehyde-containing gas or liquid.

(57) Equipment and a method for handling and/or processing fluids (e.g., gases or liquids) which contain formaldehyde are provided such that the formation of corrosive formic acid from the formaldehyde in the fluid is suppressed by fabricating surfaces and/or components of such equipment, adapted to be brought into contact with the fluid, from an alloy comprised mainly of copper and/or nickel.

EP 0 367 537 A2

## EQUIPMENT AND METHOD FOR HANDLING FORMALDEHYDE-CONTAINING GAS OR LIQUID

The present invention relates to handling a formaldehyde-containing gas or liquid in equipment which is made of a particular material. More specifically the present invention relates to handling formaldehyde in equipment in which at least those portions in contact therewith are fabricated from a metal alloy mainly comprising copper and/or nickel.

Formaldehyde has been extensively used as a starting material for synthesizing various resins, such as phenolic resin, melamine resin and urea resin, adhesives, paints, various chemicals, and medicines, or as a disinfectant a preservative, or the like.

Formaldehyde is generally produced by oxidation of methanol, and supplied in the form of an aqueous formaldehyde solution (formalin). This aqueous solution generally contains, besides unreacted methanol, minute amounts of formic acid, methylal, etc. produced by a side reaction.

In the production of formaldehyde or the process for producing the above-described substances through the use of formaldehyde, etc., various operations such as storage, transfer, concentration, purification, vaporization, condensation, and reaction of formaldehyde are conducted depending upon the purposes. In these steps, formaldehyde is handled in the form of an aqueous solution, a solution in a solvent, a liquid mixture with water or a solvent, a gaseous mixture with air, nitrogen, helium or the like, or a mixture of said solution or mixture with a main reaction product and a side reaction product.

In handling the above-described formaldehyde-containing gas or liquid, austenitic stainless steel, e.g., SUS304, SUS316 or SUS317, has been generally used in the art as a material for the equipment by taking into consideration corrosion by formaldehyde or by-products such as formic acid.

Although stainless steel is considerably superior to general steel materials etc. in corrosion resistance, the corrosion resistance at a high temperature is not yet satisfactory. Further, a side reaction wherein formic acid or the like is produced from formaldehyde (such as Cannizzaro's reaction or oxidation) is promoted probably by catalytic reaction of iron and chromium contained in stainless steel or these components dissolved out by corrosion. This brings about various problems including not only consumption of formaldehyde but also acceleration of corrosion by formic acid etc. produced by a side reaction and further necessity of removing impurities such as formic acid produced by the side reaction.

[ Summary of the Invention ]

In view of the above-described problems, the present inventors have made extensive and intensive studies on a material for constituting the equipment and, as a result, have found that an alloy mainly composed of a particular metal has very excellent resistance to corrosion caused by formaldehyde, formic acid, etc. and further a less tendency to produce formic acid etc., which has led to the completion of the present invention.

Accordingly, the present invention relates to equipment for handling a formaldehyde-containing gas or liquid, characterized in that part or the whole of the surface of said equipment to be brought into contact with a formaldehyde-containing gas or liquid is made of an alloy mainly composed of copper and/or nickel.

The present invention also provides a method of handling a formaldehyde-containing gas or liquid, wherein part or the whole of the surfaces contacted by the formaldehyde-containing gas or liquid during the handling is made of an alloy mainly composed of copper and/or nickel.

In the present invention, the material for constituting the equipment is an alloy mainly composed of copper and/or nickel, and more particularly an alloy containing at least 50% by weight in total of copper and nickel. When the total content of these components is less than 50% by weight, no sufficient effect is attained on the resistance to corrosion caused by formaldehyde, formic acid etc., and the prevention of the side reaction of formaldehyde.

The total content of these components is preferably at least 70% by weight, still preferably at least 80% by weight. In particular, an alloy containing at least 90% by weight in total of these components favorably exhibits a remarkable effect.

There is no particular limitation to the ratio of copper to nickel in the alloy, and the weight ratio of copper to nickel may be 0 to 100 : 100 to 0. However, from the viewpoint of corrosion resistance and prevention of a side reaction, it is preferred that the alloy contain both the elements even when the amount of one of the elements is very small. For example, the ratio of copper to nickel (weight ratio) is preferably 0.05 to 99.9 : 99.95 to 0.1. The weight ratio of copper to nickel is preferably 20 to 95 : 80 to 5. An alloy having a copper to nickel weight ratio of 55 to 95 : 45 to 5 is particularly preferred because it is most useful for attaining the purpose of the present invention, i.e., preventing corrosion and the side reaction, excellent in the workability and mechanical properties and also has an advantage over the above alloy in cost.

As described above, the alloy used in the present invention is mainly composed of copper and/or nickel. There is no particular limitation to alloy components other than the above-described, and use may be made of not only metallic elements such as iron, chromium, lead, zinc, aluminum, molybdenum, manganese, tungsten, and vanadium, but also silicon, carbon, sulfur, phosphorus, etc.

Specific examples of the alloy which may be used in the present invention include nickel-base alloys, such as Ni 200, Ni 201, Monel 400, Inconel 600, Inconel 625, and Hastelloy B, and copper-base alloys, such as JIS C7060, C7100, C7150, C4430, C6870, C6871, C6872, C6161, C6280, and C6301. The latter alloys are preferable as the alloy used in the present invention.

The equipment of the present invention made of the above-described material will now be described.

In the present invention, the equipment made of the above-described material is one for handling a formaldehyde containing gas liquid. Specific examples of the equipment include formaldehyde production units, storage tanks, transfer pipes, evaporators, distillation columns, heaters, condensers, coolers, and reactors. However, the equipment is not limited to these only, and the present invention can be applied to any instrument and apparatus for handling a formaldehyde-containing gas or liquid.

In the above-described equipment, formaldehyde may take various forms depending upon the purpose and function of the equipment. For example, formaldehyde may be in the form of an aqueous formaldehyde-containing solution or a solution in a solvent, a liquid mixture comprising formaldehyde and water or a solvent, or a gaseous mixture comprising formaldehyde and air, nitrogen, helium, or the like.

The above-described formaldehyde-containing gases and liquids may contain components other than the above-described, e.g., reaction products and side-reaction products of formaldehyde such as methanol, methylal, formic acid, methyl formate, and tetraoxane.

The present invention is characterized in that equipment for handling the above-described formaldehyde-containing gas and/or liquid is made of the above-described material, i. e., an alloy mainly composed of copper and/or nickel. It is not always necessary that the whole equipment is made of this material, as far as part or the whole of the surface of the equipment to be brought into contact with the formaldehyde-containing gas and/or liquid is made of the above-described material depending upon the purposes.

In particular, not only metal corrosion caused by formaldehyde and formic acid etc. produced therefrom as by-products but also a side reaction which brings about formation of formic acid etc. as by-products from formaldehyde, becomes significant as the concentration of formaldehyde or that of the formaldehyde-containing gas or liquid increases. Therefore, it is preferred that the part which is exposed to the above environment be made of the above-described material.

More specifically, it is useful to use the above-described material for the part exposed to such an environment that the formaldehyde concentration is 10 % by weight or more, and the use of the above-described material when a formaldehyde concentration is 20 % by weight more is most useful

With respect to the temperature, construction with the above-described material is useful at the part exposed to such an environment that the temperature of the formaldehyde-containing gas or liquid is 90° C or above, particularly 100° C or above.

For example, when formaldehyde is concentrated by making use of a distillation column under high temperature and high pressure conditions, formaldehyde is concentrated on a gaseous side and the formaldehyde concentration becomes high in the column top.

Therefore, in this case, part or the whole of the vicinity of the top of the distillation column and/or a condenser or a cooler for condensing and cooling a distillated coming from the column top or a receiver of the distillate, piping, etc. may be made of the above-described material. It is a matter of course that the whole apparatus including the bottom part of the column may be made of the above-described material.

As described above, the present invention relates to equipment for handling a formaldehyde-containing gas or liquid, characterized in that part or the whole of the surface of said equipment to be brought into contact with a formaldehyde-containing gas or liquid is made of an alloy mainly composed of copper and/or nickel. The present invention is applicable to and useful for any equipment for handling a formaldehyde-containing gas or liquid. More practically, a place to which the present invention is applied is determined by taking into consideration a balance between the cost (material cost) and the effectiveness, i.e., the corrosion resistance and the reduction in the amount of formaldehyde consumed by the side reaction.

As is apparent from the foregoing description and Examples, the equipment for handling a formaldehyde-containing gas or liquid according to the present invention, wherein part or the whole of the surface in contact with a formaldehyde-containing gas or liquid is made of an alloy mainly composed of copper and/or nickel, exhibits remarkable resistance to corrosion caused by formaldehyde, formic acid etc.

and further effectively suppresses occurrence of a side reaction which produces formic acid etc. from formaldehyde, which renders the equipment of the present invention very suitable as equipment for handling a formaldehyde-containing gas or liquid.

[Examples]

The present invention will now be described in more detail with reference to the following Examples which should not be construed as limiting the scope of the present invention.

Examples 1 to 6 and Comparative Examples 1 and 2

An autoclave made of glass is charged with 60 % by weight aqueous formaldehyde solution, and alloy plates (50 mm x 100 mm; two plates) falling within the scope of the present invention were immersed in the solution. The solution was heated at 100°C and 120°C for 5 days. Thereafter, methanol produced by a side reaction of formaldehyde was determined, and observation was made on the surface change in the alloy plates.

For comparison, the same evaluation was conducted also on the case where stainless steel (SUS-316) plates were used.

The results are shown in Table 1.

Table 1

| | Kind of alloy | Treating temp. (°C) | By-product concn. (wt%) | | Surface state |
|---|---|---|---|---|---|
| | | | methanol | formic acid | |
| Ex. 1 | *1 | 100 | 0.26 | 0.34 | little change |
| Ex. 2 | *2 | 100 | 0.28 | 0.37 | little change |
| Ex. 3 | *3 | 100 | 0.33 | 0.46 | little change |
| Comp. Ex. 1 | SUS316 | 100 | 0.82 | 1.20 | slight discoloration |
| Ex. 4 | *1 | 120 | 0.40 | 0.52 | little change |
| Ex. 5 | *2 | 120 | 0.42 | 0.58 | little change |
| Ex. 6 | *3 | 120 | 0.53 | 0.71 | little change |
| Comp. Ex. 2 | SUS316 | 120 | 0.21 | 1.84 | slight discoloration |
| Note: Composition: | | | | | |

*1 copper 68 %, nickel 30 %, other elements 2 %
*2 copper 87 %, nickel 10 %, other elements 3 %
*3 copper 30 %, nickel 65 %, other elements 5 %

Examples 7 and Comparative Example 3

A 1-litre round-bottomed flask made of glass was charged with 500 ml of 50% by weight aqueous formaldehyde solution, and the solution was heated under atmospheric pressure. The generated vapor was cooled in a condenser having the surface in contact with the vapor made of an alloy mainly composed of copper and nickel in a copper to nickel ratio (weight ratio) of 80 : 20. The resultant condensate was subjected to total relux into the flask. When the operation was conducted under this condition for 30 days, the formic acid concentration in the flask was 0.58% by weight. The inner surface of the condenser

4

remained uncorroded and in a mirror surface state. On the other hand, when use was made of a condenser having the surface contact with the vapor made of stainless steel (SUS 316), the concentration of formic acid in the flask after 30 days was 2.14% by weight. Further, in this case, a considerable clouding probably as a result of corrosion was observed on the inner surface.

## Claims

1. Equipment for handling a formaldehyde-containing gas or liquid, characterized in that part or the whole of the surface of said equipment to be brought into contact with a formaldehyde-containing gas or liquid is made of an alloy mainly composed of copper and/or nickel.

2. Equipment for handling a formaldehyde-containing gas or liquid according to claim 1, wherein said alloy contains 50% by weight or more in total of copper and/or nickel.

3. Equipment for handling a formaldehyde-containing gas or liquid according to claim 1 or claim 2, wherein the weight ratio of copper to nickel in said alloy is 0.05 to 99.99 : 99.95 to 0.10.

4. Equipment for handling a formaldehyde-containing gas or liquid, characterized in that part or the whole of the surface of said equipment to be brought into contact with the formaldehyde is made of an alloy containing 70% by weight or more in total of copper and nickel, with a weight ratio of copper to nickel of 20 to 95 : 80 to 5.

5. Equipment for handling a formaldehyde-containing gas or liquid, characterized in that part or the whole of the surface of said equipment to be brought into contact with the formaldehyde is made of an alloy containing 80% by weight or more in total of copper and nickel, with a weight ratio of copper to nickel of 55 to 95 : 45 to 5.

6. Equipment for handling a formaldehyde-containing gas or liquid according to any preceding claim, wherein the alloy contains one or more components selected from iron, chromium, lead, zinc, aluminium, molybdenum, manganese, tungsten, vanadium, silicon, carbon, sulphur and phosphorus.

7. Equipment for handling a formaldehyde-containing gas or liquid according to claim 6, wherein the alloy contains 2 to 5% by weight of the other component(s).

8. Equipment according to any preceding claim for concentrating an aqueous formaldehydecontaining gas or liquid, which is selected from one or more of a heater, a condenser, a cooler, a storage tank, a pressure distillation column and piping attached thereto.

9. A method of handling a formaldehyde-containing gas or liquid, wherein part or the whole of the surfaces contacted by the formaldehyde-containing gas or liquid during the handling is made of an alloy mainly composed of copper and/or nickel.

10. A method according to claim 9, wherein the temperature of said formaldehyde-containing gas or liquid is 90°C or above.